# EUROPÄISCHE PATENTSCHRIFT

(11) **EP 2 022 409 B1**
(45) Veröffentlichungstag und Bekanntmachung des Hinweises auf die Patenterteilung: **23.12.2015**
(21) Anmeldenummer: 08012820.0
(22) Anmeldetag: 16.07.2008
(51) Int. Cl.: A61B 17/00, A61F 13/20

(54) **Hämostyptikum für die minimal-invasive Operation**
Haemostyptic for minimally invasive operations
Hémostyptique pour l'opération mini-invasive

(30) Priorität: 24.07.2007 DE 102007037053
(43) Veröffentlichungstag der Anmeldung: 11.02.2009
(73) Patentinhaber: Aesculap AG, 78532 Tuttlingen (DE)
(72) Erfinder: Odermatt, Erich, 8200 Schaffhausen (CH); Wegmann, Jürgen, 78333 Stockach (DE); Blender, Bernd, 88367 Hohentengen (DE)
(74) Vertreter: Patentanwälte Ruff, Wilhelm, Beier, Dauster & Partner mbB

(56) Entgegenhaltungen:
- EP-A- 0 360 180
- WO-A-00/51499
- WO-A-03/009764
- US-A- 6 090 996
- US-A1- 2005 019 262
- US-A1- 2006 142 798
- US-B1- 6 726 696

## Beschreibung

Die Erfindung betrifft ein Hämostyptikum für die minimal-invasive Operation, insbesondere im Bauchraum. Für die Blutstillung stehen zahlreiche Methoden unterschiedlicher Art zur Verfügung. Für die Blutstillung bei minimal-invasiven Operationen sind die Möglichkeiten jedoch begrenzt. Hier werden häufig Fibrinkleber angewendet. Allerdings besteht dabei die Gefahr, dass die Kleber bei starken Blutungen aus dem Wundbereich fortgespült werden, bevor die Wunde ausreichend versiegelt und die Blutung gestillt ist.

Bewährt haben sich für die Blutstillung auch die sogenannten hämostatischen Vliese, die mit Erfolg auf dem Markt sind. Solche hämostatischen Vliese haben eine Vlies- bis Schwammstruktur und bestehen in der Regel aus Collagen oder Gelatine. Sie haben ein gutes Saugvermögen. Bei offenen Operationen werden die Vliese so lange auf die Wunde mit leichtem manuellen Druck gepresst, bis die Blutung gestillt ist. Bei starken Blutungen ist in manchen Fällen die Applikation mehrerer Vlieslagen auf die Wunde erforderlich. Die Anwendung solcher Vliese bei minimalen Operationen ist hingegen schwierig, da die Vliese zusammengerollt durch einen Trokar geschoben werden müssen. Die Vliese müssen dann im Bauchraum mit Fasszangen wieder entrollt werden. Dies erfordert ein hohes Geschick des Chirurgen. Erschwerend kommt hinzu, dass die zusammengerollten Vliese bei Kontakt mit Körperflüssigkeiten leicht verkleben und sich nicht mehr entrollen lassen. Derartige Patches auf Basis von Collagen sind in den EP 1 368 419 B1 und 1 343 542 B1 beschrieben. Sie sind zusätzlich noch mit einem Gemisch aus Fibrinogen und Thrombin beladen, um gleichzeitig die Funktion eines Fibrinklebers auszuüben.

Um das Entrollen solcher Vliesstücke bzw. Schwammstücke zu umgehen, hat die Anmelderin interne Versuche mit kleinen Viesstücken unternommen, die in ihrer Flächenausdehnung in den lichten Querschnitt eines Trokars passen. Solche tablettenförmigen Vliesstücke waren zwar leicht auszugeben und zu platzieren, es zeigte sich jedoch, dass die blutstillende Wirkung wegen der sich zwangsweise ergebenden Lücken zwischen den Einzelstücken nicht zufriedenstellend war. Es wurde deshalb nach anderen Wegen gesucht, gute blutstillende Ergebnisse bei gleichzeitiger einfacher Platzierung zu ermöglichen.

Die US 2005/0019262 A1 offenbart gegenüber biologischen Flüssigkeiten quellbare Implantate zur Befüllung von durch Biopsien entstandene Gewebekavitäten. Dieses Dokument zeigt ein Hämostyptikum in Form einer saugfähigen Scheibe aus porösem biologisch abbaubarem Material mit einem Zentrum und einer beliebigen radialen Ausdehnung.

Die WO 00/51499 A1 beschreibt eine Vorrichtung zur Blutstillung von Gefäßpunktionen auf Basis eines pfropf- bzw. zylinderförmigen Schwammes. Gegenstand der WO 03/009764 A1 sowie der US 6,090,996 sind jeweils zylinderförmige Verschlußimplantate für Gefäßpunktions- bzw. Biopsiewunden. Aus der US 2006/0142798 A1 geht ein Verschlußimplantat zum Verschluß einer Körperöffnung mit einer vorgefertigten oder erzeugbaren Öffnung hervor. Die US 6,726,696 B1 betrifft ein schichtförmig aufgebautes Gewebeverschlußimplantat mit zumindest einer Schicht eines bioverträglichen Polymers, zumindest einer Schicht eines bioverträglichen superelastischen Materials/Formgedächtnismaterials und zumindest einer Schicht eines bioverträglichen klebenden Materials. In der EP 0 360 180 A2 wird ein Verfahren zur Herstellung von Endlosbändern aus einem Kollagenschaum beschrieben.

Gegenstand der Erfindung ist ein Hämostyptikum für die minimal-invasive Operation, insbesondere im Bauchraum, gemäß Anspruch 1. Weitere Ausführungsformen sind in die abhängige Ansprüche definiert.

Durch die Erfindung ist die Möglichkeit geschaffen worden, flächige Stücke aus hämostyptischem Material in einer für die jeweilige Wunde ausreichend großen Flächenausdehnung vorzusehen, wobei durch die Art der Umformung des flächigen Materials eine einfache Ausgabe aus dem Trokar und eine leichte Platzierung auf der Wunde möglich ist. Erfindungsgemäß ist es somit vorgesehen, dass der maximale Durchmesser der Scheibe größer ist als der lichte Innendurchmesser des jeweils zur Anwendung kommenden Trokars, wobei es durch eine vorübergehende Form, insbesondere Faltung möglich ist, die Scheibe in einfacher Weise durch den Trokar auszugeben. Die Form ist insbesondere im wesentlichen rotationssymmetrisch um das Zentrum der Scheibe ausgebildet. Bevorzugt ist eine becher- oder trichterartige Form als Zwischenzustand.

Der Durchmesser der Scheibe liegt in der Regel im Bereich von 20 bis 60 mm, insbesondere 30 bis 50 mm. Ein Durchmesser von 40 mm ist eine für den normalen Gebrauch geeignete Größe der Scheibe. Die Scheibe kann einen kreisförmigen Umfang besitzen. Die Scheibe kann jedoch auch unrund sein. Eine sechseckige Außenkontur ist bevorzugt, wenn es erwünscht ist, mehrere Scheiben im wesentlichen lückenlos aneinanderreihen zu können. Auch eine ovale, eine drei- oder viereckige Außenform kann ein solches Aneinanderreihen ermöglichen. Weiterhin sind auch vieleckige Formen möglich. Der Ausdruck "im wesentlichen rotationssymmetrisch" berücksichtigt die von der Kreisform abweichenden Scheibenformen und auch die Faltenbildung am Außenrand. Erfindungsgemäß weist die Scheibe im wesentlichen radiale, linienförmige Strukturen auf, die die Überführung der Scheibe in den trichterartigen Zustand, begünstigen. Solche Strukturen sind linienförmige Strukturierungen oder Profilierungen, vorzugsweise Prägungen. Entlang der linienförmigen Prägungen kann das scheibenförmige Material in einfacher Weise gefaltet werden. Die linienförmigen Strukturen können auch bereits die Form von Faltungen besitzen. So kann die Scheibe bei einer bevorzugten Ausführungsform der Erfindung die Form eines Faltenfilters besitzen. Es ist auch möglich, die Längsrichtung der Faltung umzukehren, so dass sich Doppelfaltungen bzw. Mehrfachfaltungen ergeben.

Bei einer anderen Ausführungsform der Erfindung sind die linienförmigen Strukturen als Einschnitte ausgebildet. Solche Einschnitte, insbesondere radiale Einschnitte, erlauben eine einfache Überführung der Scheibe in eine becherartige Form. Die linienförmigen Strukturen können vom Außenrand der Scheibe bis auf einen Abstand von 5 bis 10 mm zum Zentrum der Scheibe verlaufen. Diese Ausführungsform ist insbesondere dann bevorzugt, wenn es sich bei den linienförmigen Strukturen um Einschnitte handelt, damit das Zentrum der Scheibe als flächiges Zentrum erhalten bleibt. Sind die linienförmigen Strukturen Prägungen oder Faltungen, dann können sie mit Vorteil bis ins Zentrum reichen oder kurz vor dem Zentrum enden.

Das Zentrum selbst kann geschlossen oder auch gelocht sein. Dies kann davon abhängen, in welcher Weise, d.h. mit welchen Hilfsmitteln, die Scheibe durch eine Hülse, insbesondere einen Trokar, befördert wird. Eine Lochung oder Schlitzung im Zentrum ist insbesondere dann bevorzugt, wenn mehrere oder eine Vielzahl von Scheiben hintereinander in der Hülse angeordnet sind und von einer Vorschubstange durchdrungen sind, die einzelne Scheiben nacheinander erfasst, wobei die der Wunde zugerichteten Scheiben zuerst ausgegeben werden.

Die Anzahl der linienförmigen Strukturen kann von der Art der Strukturen abhängen. So sind insbesondere dann, wenn die linienförmigen Strukturen als Einschnitte ausgebildet sind, vorzugsweise 4 bis 20, insbesondere 6 bis 12, linienförmige Strukturen vorgesehen. Die Scheiben können dann im becherartigen Zustand die Form von aufgehenden Blüten haben. Insbesondere dann, wenn die linienförmigen Strukturen die Form von Prägungen oder Faltungen besitzen, können 3 bis 40, insbesondere 3 bis 24 solcher Strukturen vorhanden sein. Im verjüngten Zustand besitzen die Scheiben dann eine plissierte Struktur oder die Form eines Faltenfilters.

Die Scheibe besitzt in der Regel eine Fläche von 3 bis 30 cm², vorzugsweise 5 bis 15 cm², insbesondere 8 bis 12 cm². In diesem Größenbereich sind die Scheiben noch leicht handhabbar und können gleichzeitig ausreichend große Wundflächen bedecken. Die Scheibendicke variiert normalerweise im Bereich von 1 mm bis 5 mm, insbesondere 2 mm bis 3 mm. Scheiben im dickeren Bereich sind auch deshalb möglich, weil das Material der Scheiben aufgrund der großen Porosität komprimierbar, insbesondere elastisch komprimierbar ist. Scheiben mit großem Durchmesser haben eher eine geringere Dicke. Auch eine von innen nach außen abnehmende Dicke kann vorgesehen sein.

Beim Material der Scheiben handelt es sich in an sich bekannter Weise um ein Protein, insbesondere um Collagen oder Gelatine. Es sind aber auch andere biologisch abbaubare Materialien möglich. So kann die Scheibe auch aus mindestens einem Polysaccharid gebildet sein, insbesondere mindestens einem aus der Gruppe Chitosan, Dextran, Hyaluronsäure, Cellulose, oxidierte Cellulose und Carboxymethylcellulose. Das Material der Scheiben kann, insbesondere wenn es aus Polysacchariden gebildet ist, faserförmig sein. Hierzu gehören auch textile Stoffe, insbesondere Gewebe oder Gewirke.

Die Scheibe kann aus einem einzigen biologisch abbaubaren Material bestehen oder aus einem Gemisch von mehreren solcher Materialien.

Es ist auch möglich, dass das Hämostyptikum einen schichtweisen Aufbau aus mindestens zwei verschiedenen und/oder verschiedenstrukturierten biologisch abbaubaren Materialien aufweist. So kann eine Schicht aus einem Protein bestehen und die andere aus mindestens einem Polysaccharid. Denkbar ist auch ein Aufbau aus unterschiedlichen Proteinen, z.B. Collagen und Gelatine. Zur Unterscheidung der Schichten kann eine Schicht mit einem Farbstoff eingefärbt werden, z.B. Riboflavin, Methylenblau.

Das poröse Material des Hämostyptikums ist mit Vorteil offenporig. Dies ermöglicht ein schnelles Aufsaugen von Körperflüssigkeit. Das poröse Material kann ferner komprimiert, insbesondere reversibel komprimiert sein, so dass es bei Kontakt mit Flüssigkeit seine ursprüngliche Schichtdicke wieder annimmt.

Das Hämostyptikum ist bei bevorzugten Ausführungsformen ein gefriergetrocknetes Material. Durch die Gefriertrocknung und insbesondere durch die Wahl des Feststoffgehaltes der der Gefriertrocknung vorgelegten Lösungen bzw. Dispersionen können Flächengewicht, Porenvolumen und Porengröße eingestellt bzw. beeinflusst werden. Der Feststoffgehalt, insbesondere Collagengehalt in den Lösungen bzw. Dispersionen liegt vorzugsweise bei 1 bis 5 Gew.-%, bezogen auf das Gesamtgewicht der Lösungen bzw. Dispersionen. Die Dichte des porösen Materials liegt vorzugsweise bei 10 bis 50 g/dm³, insbesondere 20 bis 40 g/dm³, bevorzugt 25 bis 35 g/dm³. Das poröse Material des Hämostyptikums hat mit Vorteil ein Porenvolumen von > 90 Vol.-%, insbesondere im Bereich von 96 bis 99 Vol.-%. In der Regel liegt das Porenvolumen bei 96 bis 98 Vol.-%. Das Flächengewicht des porösen Materials liegt normalerweise bei 5 bis 40 mg/cm², vorzugsweise 10 bis 20 mg/cm², insbesondere bei ca. 10 mg/cm². Das Flächengewicht hängt von der Schichtdicke des Materials ab.

Sofern ein geringeres Porenvolumen erwünscht ist, kann der Gehalt an biologisch abbaubarem Material in der zur Gefriertrocknung bestimmten Lösung bzw. Dispersion so weit wie möglich entsprechend erhöht werden. Als Alternative bietet sich die bereits erwähnte Komprimierung an. So kann das Porenvolumen durch Komprimierung auf 60 bis 95 Vol.-% eingestellt werden.

Das poröse Material des Hämostyptikums ist trotz seiner Leichtigkeit geschmeidig und biegsam. Es ist mit Vorteil bis zu einer maximalen Stempeldurchpresskraft von 8 bis 15 Newton, insbesondere 10 bis 13 Newton, stabil.

Das Flüssigkeitsaufnahmevermögen des erfindungsgemäßen Hämostyptikums ist enorm hoch. Es liegt mit Vorteil beim 15- bis 60-fachen, insbesondere 25- bis 60-fachen, vorzugsweise 30- bis 60-fachen, des Eigengewichts des Hämostyptikums. Das große Flüssigkeitsaufnahmevermögen ermöglicht es, große Flüssigkeitsmengen zu binden. Hinzu kommt, dass die Bindung der Körperflüssigkeiten sehr schnell von statten geht. So kann das poröse Material des Hämostyptikums innerhalb eines Zeitraums von weniger als 100 Sekunden, insbesondere weniger als 60 Sekunden, vollständig mit Wasser benetzbar sein. Ähnliches gilt für Körperflüssigkeiten.

Mit besonderem Vorteil ist für das Hämostyptikum nur eine Art von Eiweiß vorgesehen, d.h. es ist frei von anderen Eiweißbestandteilen. Als Eiweißbestandteil ist Collagen bevorzugt. Collagen kann in Form von einem Typ oder mehreren Typen der Typen I bis IV vorliegen.

Als Collagen ist vorzugsweise xenogenes Collagen vorgesehen, insbesondere solches porcinen, bovinen oder equinen Ursprungs.

Mit besonderem Vorteil kann das Hämostyptikum im porösen Material auch noch mindestens eine organische Säure, vorzugsweise mindestens eine mehrwertige organische Säure enthalten. Solche Säuren bewirken bei der Gefriertrocknung eine membran- oder plättchenartige Struktur des biologisch abbaubaren Materials, die zu einer Vergrößerung der inneren Oberfläche führt.

In einer bevorzugten Ausführungsform besitzt das Hämostyptikum in Wasser bzw. bei Kontakt mit Wasser einen pH-Wert < 4. Vorzugsweise besitzt das Hämostyptikum in Wasser bzw. bei Kontakt mit Wasser einen pH-Wert zwischen 3,0 und 3,5.

Die Porengröße des Hämostyptikums liegt vorzugsweise unter 500 µm, insbesondere unter 300 µm, bevorzugt unter 100 µm. Die kleine Porengröße des Hämostyptikums ermöglicht das Auftreten größerer Kapillarkräfte, wodurch ein größeres Aufnahmevermögen für Flüssigkeiten, insbesondere Körperflüssigkeiten, vorzugsweise Blut, resultiert.

In einer weiteren Ausführungsform verfügt das Hämostyptikum über flexible und insbesondere stabile Eigenschaften. Vorzugsweise ist der Säuregehalt im Hämostyptikum so hoch, dass diese Eigenschaften nicht beeinträchtigt sind. Der Gehalt an Säure im Hämostyptikum liegt bevorzugt zwischen 1 und 25 Gew.-%, insbesondere zwischen 2 und 15 Gew.-%, besonders bevorzugt zwischen 3 und 10 Gew.-%.

In einer weitergehenden Ausführungsform ist die wasserlösliche bioverträgliche organische Säure eine nicht flüchtige Säure. Bevorzugt ist die Säure eine aliphatische Säure. Beispielsweise kann die Säure eine Kohlenstoffkette mit 1 bis 6 Kohlenstoffatomen, vorzugsweise 3 bis 6 Kohlenstoffatomen, aufweisen. Bei der Säure kann es sich um eine oligofunktionelle, insbesondere bi- oder trifunktionelle, Säure handeln. Die Säure kann insbesondere eine mehrwertige, beispielsweise zweiwertige, Säure sein. Bevorzugt handelt es sich bei der Säure um eine Hydroxycarbonsäure, insbesondere mehrwertige Hydroxycarbonsäure. Beispielsweise kann es sich bei der Säure auch um eine Zuckersäure handeln.

Bei der wasserlöslichen bioverträglichen organischen Säure des Hämostyptikums handelt es sich vorzugsweise um eine Säure aus der Gruppe, umfassend Zitronensäure, Weinsäure, Ascorbinsäure, Apfelsäure, Gluconsäure, Schleimsäure, Glutarsäure und Adipinsäure. Äpfelsäure (Apfelsäure) ist besonders bevorzugt.

Die wasserlösliche bioverträgliche organische Säure des Hämostyptikums kann insbesondere in Form eines Salzes vorliegen. Bei der Salzverbindung handelt es sich vorzugsweise um ein Calciumsalz der Säure, beispielsweise um Calciumcitrat. Die Verwendung eines Calciumsalzes ist besonders vorteilhaft, da die Blutstillung durch Calciumionen beschleunigt werden kann.

Gegenstand der Erfindung ist auch das Hämostyptikum in verpacktem Zustand. Dabei liegt die Scheibe vorzugsweise bereits in trichterförmiger Form, vor. Mit anderen Worten kann das Hämostyptikum, insbesondere im radial verjüngten Zustand, in einer Verpackung verpackt sein. Die Verpackung kann eine Einmalverpackung sein. Die Verpackung ist vorzugsweise steril. Als Verpackung ist vorzugsweise eine hülsenförmige Verpackung vorgesehen. Diese kann einen 5 mm bis 13 mm großen Innendurchmesser besitzen. In einer solchen Hülse, die auch als Reduzierhülse bezeichnet wird, kann mindestens eine Scheibe vorgelegt sein. Es können auch axial hintereinander zahlreiche radial verjüngte Scheiben vorliegen. Als Verpackung kann eine Hülse eines Trokars vorgesehen sein. Die Hülse kann in einen Trokar einschiebbar sein. Die Hülse kann auch so ausgestaltet sein, dass die mindestens eine Scheibe aus der Hülse in einfacher Weise in einen Trokar überführbar ist. Erfindungsgemäß ist es besonders bevorzugt, dass mehrere Scheiben in radial verjüngtem Zustand in axialer Anordnung in einer Ausgabehülse gelagert sind, der vorzugsweise ein Applikator zur einzelnen Ausgabe der Scheiben zugeordnet ist.

Weitere Merkmale und Vorteile der Erfindung ergeben sich aus der nachfolgenden Beschreibung von bevorzugten Ausführungsformen der Erfindung anhand der Zeichnung und den Beispielen in Verbindung mit den Unteransprüchen.

In der Zeichnung zeigen
- Figur 1:: eine Ausführungsform des erfindungsgemäßen Hämostyptikums in flächigem Zustand;
- Figur 2:: die Ausführungsform nach Figur 1 in becherförmig , nicht Teil der Erfindung, gefalteten Zustand;
- Figur 3:: eine andere Ausführungsform der Erfindung in mehrfacher Form in einem Magazin;
- Figur 4:: eine weitere Ausführungsform der Erfindung in mehrfacher Form in einem Magazin und
- Figur 5:: eine weitere Ausführungsform der Erfindung.

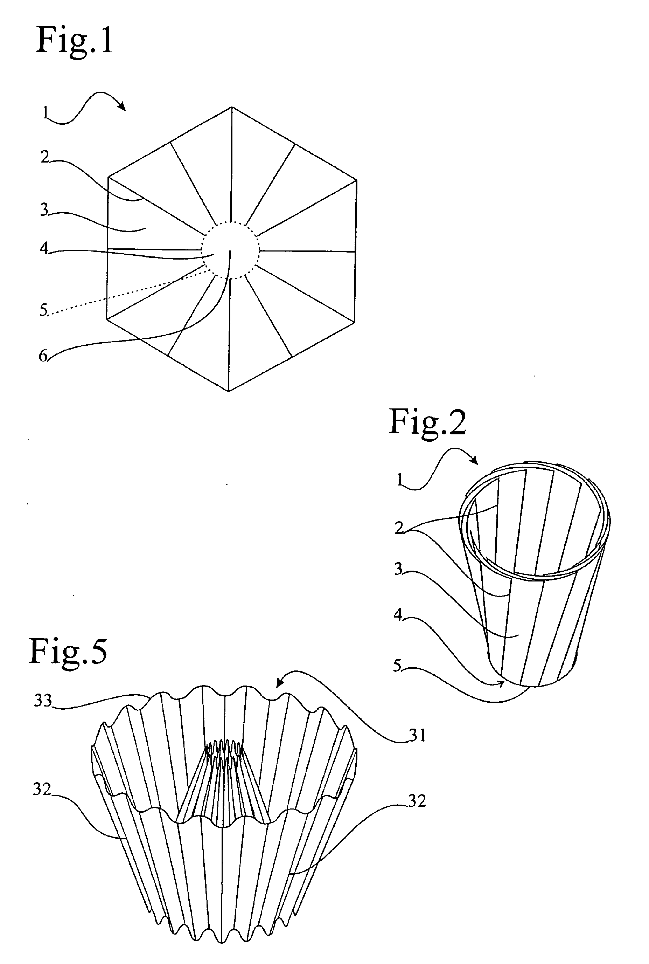

Bei der in den Figuren 1 und 2 dargestellten Ausführungsform der Erfindung ist ein Hämostyptikum 1 in Form einer sechseckigen Scheibe mit einem maximalen Durchmesser von 50 mm und einer Dicke von 2 mm vorgesehen. Die Scheibe besteht aus gefriergetrocknetem Collagen. Die Scheibe 1 ist durch zwölf radiale Linien 2 in zwölf Segmente 3 unterteilt.

Die radialen Linien sind Einschnitte, die von den Ecken des Sechsecks und zwischen den Ecken ausgehen und bis in einen zentralen Bereich 4 der Scheibe 1 reichen, der einen Durchmesser von ca. 8 mm besitzt und in dem das Zentrum 6 liegt.

Das gefriergetrocknete Collagenmaterial der Scheibe 1 ist weich und flexibel und lässt sich leicht biegen. Am Übergang vom zentralen Bereich 4 in die Segmente 3 ist eine Umrisslinie 5 des zentralen Bereichs mit einer Prägung versehen. Das Hämostyptikum hat somit das Aussehen einer Korbblüte mit Blütenblättern, die um den mittleren Korb angeordnet sind. Der Bereich 4 erleichtert bei der Anwendung die Fixierung der Scheibe 1 auf der Gewebeoberfläche z.B. mit einem stabartigen Instrument bzw. einer Vorschubstange.

Wie in Figur 2 zu sehen, sind die Segmente 3 entlang der Umrisslinie 5 in einer Richtung abgebogen und überlappen sich am Außenrand gegenseitig. Dadurch besitzt das Hämostyptikum die Form eines Bechers bzw. einer aufgehenden Blüte. In diesem Zustand beträgt der wirksame Außendurchmesser des Hämostyptikums ca. 20 mm, wobei sich dieser Durchmesser beim Einschieben in ein Rohr von ca. 10 mm Innendurchmesser noch weiter verjüngen lässt. Das Hämostyptikum kann auf diese Weise bei einer minimal-invasiven Operation zur Blutstillung durch einen Trokar durchgeschoben und durch Wiederausbreiten der Segmente ohne Probleme in die flächige Form zurückgeführt werden. Durch Aneinanderlagerung der sechseckigen Scheiben können größere Flächen lückenlos bedeckt werden.

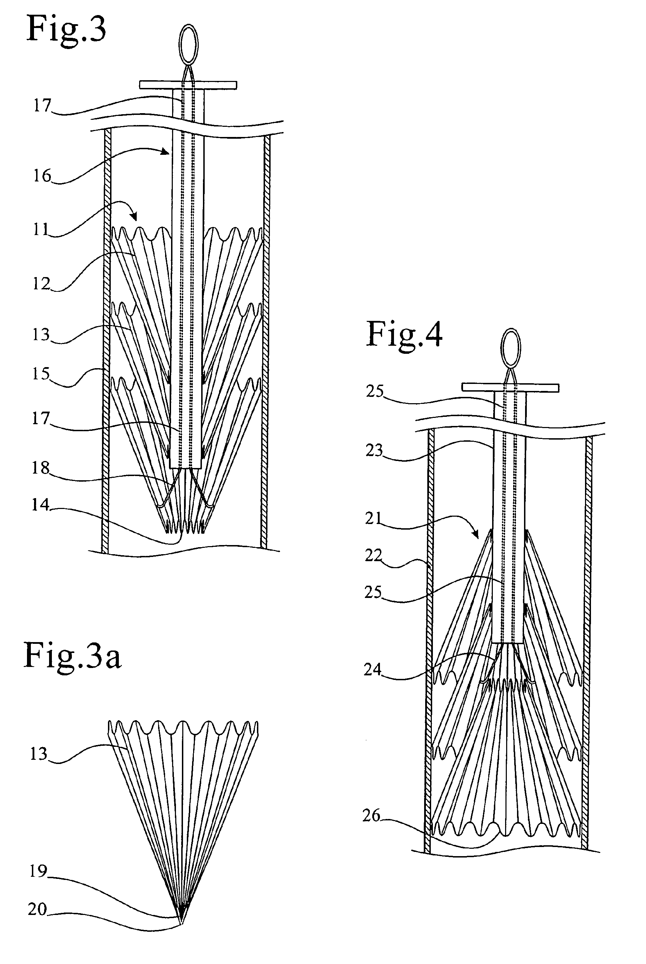

Bei der Ausführungsform nach Figur 3 sind kreisrunde Scheiben 11 mit einem Durchmesser von 40 mm nach Art eines Schirmes oder eines Faltenfilters gefaltet. Hierzu weist die Scheibe radiale Prägungslinien 12 auf, entlang derer das gefriergetrocknete Collagenmaterial gefaltet ist, und zwar abwechselnd nach vorne und nach hinten. Die dadurch gebildeten Falten 13 erlauben durch Zusammenschieben eine starke Verringerung des Außendurchmessers der Scheibe, wobei in Figur 3 aus Gründen der besseren Übersichtlichkeit nur ein teilweises Zusammenschieben dargestellt ist. Die nicht dargestellte Spitze der gefalteten Scheibe ist abgeschnitten, so dass hier im Zentrum ein axiales Durchgangsloch 14 vorhanden ist. Anstelle des Loches können im Zentrum 19 auch kurze radiale Schlitze 20, z.B. Kreuzschlitze, vorgesehen sein, so dass kein Materialverlust vorliegt (Fig. 3a).

Bei der dargestellten Ausführungsform sind drei Hämostyptika in axialer Richtung hintereinander in einem Rohr 15 in gefaltetem Zustand angeordnet, wobei die Hämostyptika teilweise ineinander geschoben sind. Durch die Löcher 14 ist ein Applikator 16 geschoben, der im Inneren des vordersten Hämostyptikums endet. Der Applikator 16 ist rohrförmig ausgebildet. Im Inneren des Applikators ist eine Vorschubstange 17 angeordnet, die an ihrem vorderen Ende drei radial gespreizte Federarme 18 besitzt. Die Federarme liegen an der Innenseite der gefalteten Scheibe an, so dass die Scheibe durch die Vorschubstange 17 aus dem Rohr 15 herausgeschoben werden kann. Ist das Rohr 15 ein Trokar oder ein in einen Trokar einführbares rohrförmiges Magazin, dann können auf diese Weise die Hämostyptika nacheinander durch die Bauchwand auf die Wunde ausgegeben werden, auf der Blutstillung erfolgen soll.

Nach Ausgabe des ersten Hämostyptikums kann die Vorschubstange 17 im Applikator 16 zurückgezogen werden, wobei die Federarme 18 mit eingezogen werden und sich dabei aneinander legen. Der Applikator wird dann insgesamt etwas zurückgezogen, so dass sein Ende wiederum im zweiten jetzt vordersten Hämostyptikum zu liegen kommt. Die Vorschubstange kann dann zusammen mit den Federarmen wieder aus dem Applikator herausgeschoben werden, wobei sich der Vorgang der Applikation wiederholt und nach dem zweiten auch weitere Hämostyptika in gleicher Weise ausgegeben werden können.

Bei der Ausführungsform nach Figur 4 sind kreisrunde Scheiben 21 in gleicher Weise ausgebildet wie bei der Ausführungsform nach Figur 3. Sie sind jedoch in umgekehrter Richtung in einem Magazinrohr 22 gelagert. Es ist wiederum ein Applikator 23 vorgesehen, der gleich ausgebildet ist wie bei der Ausführungsform nach Figur 3. Federarme 24 einer Vorschubstange 25 greifen jedoch nicht wie bei Figur 3 an der Innenseite sondern an der Außenseite des konisch verjüngten Bereichs der gefalteten Scheiben an. Durch die Federarme kann die gefaltete Scheibe aus dem Magazinrohr 22 wiederum herausgeschoben werden, wobei diesmal der gefaltete Außenrand der Scheibe 21 zuerst aus dem Rohr austritt. Auch hier können beliebig viele Hämostyptika im Magazinrohr gelagert werden. Die Vorschubbewegung des Applikators 23 erfolgt entsprechend.

Bei den Ausführungsformen nach Figuren 3 und 4 können das Magazinrohr, die Scheiben der Hämostyptika und der Applikator jeweils als sterile Einwegverpackung vorgesehen sein, die dann zum Einsatz kommt, wenn bei einer minimal-invasiven Operation eine Blutstillung erfolgen soll.

Bei der Ausführungsform nach Figur 5 liegt ein Hämostyptikum in Form einer doppelt gefalteten Scheibe 31 vor. Die Scheibe besitzt eine Grundfaltung, die der der Ausführungsformen nach den Figuren 3 und 4 entspricht. Es sind wiederum radiale Prägungen zur Ausbildung der Faltung vorgesehen, wobei der Außenrand 33 der Scheibe etwa ab der Mitte der radialen Prägungen in entgegengesetzter Richtung umgeklappt ist, so dass sich eine doppelte, übereinander liegende Faltung der Scheibe ergibt. Die Faltung entspricht somit der eines Schirmes, der zur Verkürzung der Schirmlänge in sich umklappbar ist.

### Beispiel 1:

In 660 ml Reinstwasser (MilliQ-Wasser, Firma Millipor, Deutschland) werden 33 g Collagen gequollen. Das gequollene Collagen wird für etwa 20 Minuten in einem Lösungsmittelgemisch aus 1155 ml Reinstwasser und 165 ml Isopropanol suspendiert. Danach werden in ca. 1320 ml Suspensionslösung ungefähr 1,32 g Äpfelsäure (0,1 Gew.-%, bezogen auf das Gesamtgewicht der Collagensuspension) gelöst. Anschließend werden jeweils 130 g der Suspension auf Lyophilisationsschalen mit einer Grundfläche von ca. 165 cm² gegossen, bei - 40 °C eingefroren und lyophilisiert. Als Produkt werden rechteckige Collagenplatten erhalten. Die Dicke dieser Platten variiert mit der Füllmenge der Lyophilisationsschalen. Normalerweise werden Platten mit einer Schichtdicke von 1 bis 2 mm hergestellt, dickere Platten können durch Kompression auf etwa die Hälfte verdichtet werden. Gleichzeitig mit der Verdichtung können radiale Prägelinien oder Stanzlinien ausgebildet werden und auch die Außenform der Scheiben in gewünschter Form und Größe ausgestanzt werden. Gleichzeitig können auch zentrale Lochungen oder zentrale radiale Schlitzungen vorgenommen werden. Die gewünschten Linien können auch ohne dass eine Kompression vorgesehen ist, gebildet werden, wobei im Falle von Prägelinien auch gleichzeitig eine Faltung vorgeformt werden kann.

### Beispiel 2:

### Minimal invasive Blutstillung im Schwein

Mit Hilfe eines Throkars (Innendurchmesser 10 mm) wurde minimal invasiv ein Zugang zur Milz gelegt. Auf der Milz wurde eine 2 x 2 cm große kapsuläre Blutung induziert. Über den Throkar wurden kreisrunde Collagenpads mit einem Durchmesser von 10 mm und einer Dicke von 4 mm auf die blutende Wunde appliziert. Die Blutung konnte trotz des Aufbringens von mehreren Lagen erst nach 10 Minuten gestillt werden. Eine analoge kapsuläre Verletzung der Milz konnte durch Auflage eines kreisrunden Bechers gemäß der Erfindung mit einem Außendurchmesser von ca. 30 mm und 6 Einschnitten (10 mm) innerhalb von 90 Sekunden gestillt werden. Die Dicke des Collagenmaterials betrug 4 mm.

An der Leber wurden ebenfalls über einen minimal invasiven Zugang 2 x 2 cm große kapsuläre Blutungen induziert. Für diese Versuche wurden Collagenpads bzw. Collagenbecher mit Äpfelsäurezusatz (Herstellung gemäß Beispiel 1) verwendet. Die kreisrunden Collagenpads (Durchmesser 10 mm, Dicke 4 mm) konnten die Blutung trotz Auflagen mehrerer Layers innerhalb von 10 Minuten nicht stillen, während analoge Leberverletzungen durch Auflage von kreisrunden Bechern mit einem Außendurchmesser von ca. 30 mm und 6 Einschnitten (10 mm) innerhalb von 180 Sekunden gestillt werden konnte.

## Patentansprüche

1. Hämostyptikum für die minimal-invasive Operation, insbesondere im Bauchraum, in Form einer saugfähigen Scheibe (1, 11, 21, 31) aus porösem biologisch abbaubarem Material mit einem Zentrum (6) und einer radialen Ausdehnung von mindestens 10 mm vom Zentrum, wobei die Scheibe derart ausgebildet ist, dass sie eine trichterartige Form aufweist, in welcher sie durch einen Tubus (15) in den Körper einführbar ist und aus dem sie wieder in den flächigen Zustand überführbar ist, wobei die Scheibe (1, 11, 21, 31) im wesentlichen radiale, linienförmige Strukturen (2, 12, 32) aufweist, die die Überführung in die trichterförmige Form als Zwischenzustand begünstigen, und die linienförmigen Strukturen Profilierungen sind.

2. Hämostyptikum nach Anspruch 1, **dadurch gekennzeichnet, dass** die Profilierungen Prägungen (12) sind.

3. Hämostyptikum nach Anspruch 1 oder Anspruch 2, **dadurch gekennzeichnet, dass** die linienförmigen Strukturen Faltungen (13) sind.

4. Hämostyptikum nach einem der vorhergehenden Ansprüche, **dadurch gekennzeichnet, dass** die linienförmigen Strukturen Einschnitte (2) sind.

5. Hämostyptikum nach einem der vorhergehenden Ansprüche, **dadurch gekennzeichnet, dass** die linienförmigen Strukturen vom Außenrand der Scheibe bis auf einen Abstand von 5 bis 10 mm vom Zentrum (6) der Scheibe verlaufen.

6. Hämostyptikum nach Anspruch 2 oder 3, **dadurch gekennzeichnet, dass** die Prägungen (12) und/oder Faltungen (13) im wesentlichen bis zum Zentrum der Scheibe verlaufen, wogegen Einschnitte (2) vor dem Zentrum (6) enden.

7. Hämostyptikum nach einem der vorhergehenden Ansprüche, **dadurch gekennzeichnet, dass** die Scheibe (11, 21) einen im wesentlichen kreisförmigen Umfang besitzt.

8. Hämostyptikum nach einem der Ansprüche 1 bis 6, **dadurch gekennzeichnet, dass** die Scheibe (1) einen im wesentlichen polygonalen Umfang besitzt.

9. Hämostyptikum nach Anspruch 8, **dadurch gekennzeichnet, dass** die Scheibe (1) im wesentlichen eine Umfangslinie eines Dreiecks, eines Vierecks oder vorzugsweise eines Sechsecks besitzt.

10. Hämostyptikum nach einem der vorhergehenden Ansprüche, **dadurch gekennzeichnet, dass** die Scheibe (11) ein mittiges Loch (14) oder eine mittige Schlitzung (20) aufweist.

11. Hämostyptikum nach einem der vorhergehenden Ansprüche, **dadurch gekennzeichnet, dass** 4 bis 20, insbesondere 6 bis 12 linienförmige Strukturen, insbesondere in Form von Einschnitten (2) vorgesehen sind.

12. Hämostyptikum nach Anspruch 2 oder 3, **dadurch gekennzeichnet, dass** 3 bis 40, insbesondere 3 bis 24 linienförmige Strukturen in Form von Prägungen (12) oder Faltungen (13) vorgesehen sind.

13. Hämostyptikum nach einem der vorhergehenden Ansprüche, **dadurch gekennzeichnet, dass** die Scheibe eine Fläche von 3 bis 30 cm², vorzugsweise 5 bis 15 cm², insbesondere 8 bis 12 cm², besitzt.

14. Hämostyptikum nach einem der vorhergehenden Ansprüche, **dadurch gekennzeichnet, dass** die Scheibe eine Dicke von 1 bis 5 mm, insbesondere 2 bis 3 mm, besitzt.

15. Hämostyptikum nach einem der vorhergehenden Ansprüche, **dadurch gekennzeichnet, dass** es aus mindestens einem Protein, insbesondere Kollagen oder Gelatine gebildet ist.

16. Hämostyptikum nach einem der vorhergehenden Ansprüche, **dadurch gekennzeichnet, dass** es aus mindestens einem Polysaccharid, insbesondere aus der Gruppe Chitosan, Hyaluronsäure, Dextran, Cellulose, oxidierte Cellulose, Carboxymethylcellulose, gebildet ist, insbesondere besteht.

## Claims

1. A haemostatic device for minimally invasive surgery, in particular in the abdominal cavity, which device is in the form of an absorbent disc (1, 11, 21, 31) made of a porous biodegradable material with a centre (6) and a radial extension of at least 10 mm from the centre, wherein the disc is formed in such a way that it has a cone-type shape in which it can be introduced into the body through a tube (15) and from which it can be reconverted in a flat condition, wherein the disc (1, 11, 21, 31) has essentially radial linear structures (2, 12, 32) that facilitate conversion into the cone-type shape intermediate state, and the linear structures are profilings.

2. The haemostatic device according to Claim 1, **characterized in that** the profilings are embossments (12).

3. The haemostatic device according to Claim 1 or Claim 2, **characterized in that** the linear structures are folds (13).

4. The haemostatic device according to any one of the preceding Claims, **characterized in that** the linear structures are incisions (2).

5. The haemostatic device according to any one of the preceding Claims, **characterized in that** the linear structures extend from the outer edge of the disc up to a distance of 5 to 10 mm to the centre (6) of the disc.

6. The haemostatic device according to Claim 2 or 3, **characterized in that** the embossments (12) and/or folds (13) essentially extend up to the centre of the disc, whereas incisions (2) end before reaching the centre (6).

7. The haemostatic device according to any one of the preceding Claims, **characterized in that** the disc (11, 21) has an essentially circular circumference.

8. The haemostatic device according to any one of Claims 1 to 6, **characterized in that** the disc (1) has an essentially polygonal circumference.

9. The haemostatic device according to Claim 8, **characterized in that** the disc (1) has an essentially triangular, rectangular or preferably hexagonal circumferential line.

10. The haemostatic device according to any one of the preceding Claims, **characterized in that** the disc (11) has a central hole (14) or a central slotting (20).

11. The haemostatic device according to any one of the preceding Claims, **characterized in that** 4 to 20, in particular 6 to 12 linear structures, in particular in the form of incisions (2) are provided.

12. The haemostatic device according to Claim 2 or 3, **characterized in that** 3 to 40, in particular 3 to 24 linear structures in the form of embossments (12) or folds (13) are provided.

13. The haemostatic device according to any one of the preceding Claims, **characterized in that** the disc has a surface area of 3 to 30 cm², preferably 5 to 15 cm², in particular 8 to 12 cm².

14. The haemostatic device according to any one of the preceding Claims, **characterized in that** the disc has a thickness of 1 to 5 mm, in particular 2 to 3 mm.

15. The haemostatic device according to any one of the preceding Claims, **characterized in that** it is composed of at least one protein, in particular collagen or gelatine.

16. The haemostatic device according to any one of the preceding Claims, **characterized in that** it is composed of, in particular consisting of, at least one polysaccharide, in particular from the group consisting of chitosan, hyaluronic acid, dextran, cellulose, oxidized cellulose and carboxymethylcellulose.

## Revendications

1. Hémostyptique pour l'opération mini-invasive, en particulier dans la cavité abdominale, en forme d'un disque absorbant (1, 11, 21, 31) en matériau poreux biologiquement dégradable comportant un centre (6) et une extension radiale d'au moins 10 mm à partir du centre, le disque étant conçu de manière à présenter une forme d'entonnoir, qui peut être introduit dans le corps par un tube (15) et à partir duquel il peut être remis en l'état plat, le disque (1, 11, 21, 31) présentant des structures linéaires (2, 12, 32) essentiellement radiales, qui favorisent la conversion en la forme d'entonnoir en tant qu'état intermédiaire, et les structures linéaires étant des profilages.

2. Hémostyptique selon la revendication 1, **caractérisé en ce que** les profilages sont des gaufrages (12).

3. Hémostyptique selon la revendication 1 ou la revendication 2, **caractérisé en ce que** les structures linéaires sont des pliages (13).

4. Hémostyptique selon l'une quelconque des revendications précédentes, **caractérisé en ce que** les structures linéaires sont des incisions (2).

5. Hémostyptique selon l'une quelconque des revendications précédentes, **caractérisé en ce que** les structures linéaires s'étendent du bord extérieur du disque jusqu'à une distance de 5 à 10 mm du centre (6) du disque.

6. Hémostyptique selon la revendication 2 ou 3, **caractérisé en ce que** les gaufrages (12) et/ou les pliages (13) s'étendent essenttiellement jusqu'au centre du disque, tandis que les incisions (2) se terminent avant le centre (6).

7. Hémostyptique selon l'une quelconque des revendications précédentes, **caractérisé en ce que** le disque (11, 21) présente une circonférence essentiellement circulaire.

8. Hémostyptique selon l'une quelconque des revendications 1 à 6, **caractérisé en ce que** le disque (1) présente une circonférence essentiellement polygonale.

9. Hémostyptique selon la revendication 8, **caractérisé en ce que** le disque (1) présente essentiellement une ligne circonférentielle d'un triangle, d'un quadrilatère ou de préférence d'un hexagone.

10. Hémostyptique selon l'une quelconque des revendications précédentes, **caractérisé en ce que** le disque (11) comporte un trou central (14) ou une fente centrale (20).

11. Hémostyptique selon l'une quelconque des revendications précédentes, **caractérisé en ce que** sont prévues 4 à 20, en particulier 6 à 12 structures linéaires, en particulier en forme d'incisions (2).

12. Hémostyptique selon la revendication 2 ou 3, **caractérisé en ce que** sont prévues 3 à 40, en particulier 3 à 24 structures linéaires en forme de gaufrages (12) ou de pliages (13).

13. Hémostyptique selon l'une quelconque des revendications précédentes, **caractérisé en ce que** le disque présente une surface de 3 à 30 cm², de préférence 5 à 15 cm², en particulier 8 à 12 cm².

14. Hémostyptique selon l'une quelconque des revendications précédentes, **caractérisé en ce que** le disque présente une épaisseur de 1 à 5 mm, en particulier 2 à 3 mm.

15. Hémostyptique selon l'une quelconque des revendications précédentes, **caractérisé en ce qu'**il est constitué d'au moins une protéine, en particulier de collagène ou de gélatine.

16. Hémostyptique selon l'une quelconque des revendications précédentes, **caractérisé en ce qu'**il est constitué à base d'au moins, en particulier consiste en au moins, un polysaccharide, en particulier choisi dans le groupe formé par le chitosane, l'acide hyaluronique, le dextrane, la cellulose, la cellulose oxydée, la carboxyméthylcellulose.
